# EUROPÄISCHE PATENTSCHRIFT

(11) **EP 3 244 817 B1**
(45) Veröffentlichungstag und Bekanntmachung des Hinweises auf die Patenterteilung: **28.11.2018**
(21) Anmeldenummer: 16718343.3
(22) Anmeldetag: 22.04.2016
(51) Int. Cl.: A61B 17/70

(54) **VORRICHTUNG ZUM DURCHFÜHREN EINER DISTRAKTION ODER EINER KOMPRESSION VON WIRBELKÖRPERN BEI EINER WIRBELSÄULENOPERATION**
DEVICE FOR CARRYING OUT A DISTRACTION OR A COMPRESSION OF VERTEBRAL BODIES DURING SPINAL SURGERY
PROCÉDÉ POUR LA RÉALISATION D'UN ÉCARTEMENT OU D'UNE COMPRESSION DES VERTÈBRES DANS LE CAS D'UNE INTERVENTION CHIRURGICALE SUR LA COLONNE VERTÉBRALE

(30) Priorität: 29.06.2015 DE 102015212050
(43) Veröffentlichungstag der Anmeldung: 22.11.2017
(73) Patentinhaber: Silony Medical International AG, 8500 Frauenfeld (CH)
(72) Erfinder: OHNMACHT, Timo, 78736 Trichtingen (DE); HEUER, Frank, 70794 Filderstadt (DE)
(74) Vertreter: DREISS Patentanwälte PartG mbB
(86) Internationale Anmeldenummer: PCT/EP2016/059063
(87) Internationale Veröffentlichungsnummer: WO 2017/001080

(56) Entgegenhaltungen:
- EP-A1- 2 305 154
- DE-U1- 8 712 943
- US-A1- 2014 039 557

## Beschreibung

Die Erfindung betrifft eine Vorrichtung zum Durchführen einer Distraktion oder einer Kompression von Wirbelkörpern bei einer, insbesondere minimalinvasiven, Wirbelsäulenoperation, mit einem ersten und einem zweiten, insbesondere zumindest abschnittsweise hülsenförmigen, Verlängerungsteil mit einer jeweiligen Längsrichtung, welches jeweils an einem Knochenanker zwar lösbar jedoch starr und drehfest festlegbar ist, wobei die Knochenanker jeweils in einen der zu behandelnden Wirbelkörper einzubringen oder bereits eingebracht sind.

In der modernen Wirbelsäulenchirurgie, insbesondere der minimal invasiven Wirbelsäulenchirurgie, wo mit hülsenförmigen Verlängerungsteilen der Knochenanker, sogenannten Extendern, gearbeitet wird, stellt sich häufig die Aufgabe, dass in der Regel benachbarte Wirbelkörper voneinander weg (Distraktion) oder aufeinander zu (Kompression) bewegt werden müssen. Die neue Position kann dann über Osteosynthesevorrichtungen mit Korrekturstäben fixiert werden. Hierfür gibt es im Stand der Technik teils aufwändig konstruierte Instrumente, welche die beiden Verlängerungsteile, die mit den in die Wirbelkörper eingebrachten Knochenankern starr verbunden sind, verstellbar zueinander verbinden. Die vorbekannten Vorrichtungen sind aufwändig herstellbar und komplex bedienbar.

Aus US 2014/0039557 A1 ist eine Vorrichtung der eingangs genannten Art bekannt. Bei dieser Vorrichtung ist bei einem der hülsenförmigen Verlängerungsteile ein in der Längsrichtung verschiebliches Lagerteil vorgesehen, gegen welches das andere hülsenförmige Verlängerungsteil zur Ausbildung einer Lagerstelle anlegbar ist. Das verschiebliche Lagerteil ist durch Aufklipsen eines Aufsatzteils vergrößerbar.

US 2014/0277151 A1 offenbart ebenfalls eine Vorrichtung der eingangs genannten Art mit einem kappenförmigen, auf eines der hülsenförmigen Verlängerungsteile aufsteckbaren und gegebenenfalls in der Längsrichtung bezüglich des hülsenförmigen Verlängerungsteils verschieblichen Lagerteils, welches U-förmige oder flügelförmige Ansätze oder Arme aufweist, die sich in Querrichtung nach außen erstrecken und gegen welche das andere hülsenförmige Verlängerungsteil anlegbar ist, um eine Schwenklagerstelle zu bilden. Zur Adaptierung verschiedener Querabstände der hülsenförmigen Verlängerungsteile werden verschiedene Lagerteile unterschiedlicher Quererstreckung verwendet oder ein Lagerteil, bei dem ein Arm in Querrichtung längenverstellbar ausgebildet ist, wobei ein zahnstangenartiger Verriegelungsmechanismus zum Einsatz kommen kann.

Hiervon ausgehend liegt der vorliegenden Erfindung die Aufgabe zugrunde, eine Vorrichtung der eingangs genannten Art zu schaffen, die weniger komplex aufgebaut ist als vorbekannte Vorrichtungen, die einfach bedienbar und auf wirtschaftliche Weise herstellbar ist.

Diese Aufgabe wird durch eine Vorrichtung der eingangs genannten Art gelöst, die gekennzeichnet ist durch ein erstes exzentrisches Lagerteil und ein zweites Gegenlagerteil, die auf einander entsprechender Position in der Längsrichtung an dem ersten bzw. an dem zweiten Verlängerungsteil positionierbar oder positioniert sind, wobei das erste exzentrische Lagerteil ein zusätzliches zu dem ersten Verlängerungsteil bewegbares Bauteil ist und das zweite Gegenlagerteil ein zu dem zweiten Verlängerungsteil zusätzliches bewegbares Bauteil ist oder von dem zweiten Verlängerungsteil selbst gebildet ist, und dadurch dass zumindest das exzentrische Lagerteil relativ zu dem ersten Verlängerungsteil um dessen Längsrichtung drehbar ist, so dass aufgrund der Exzentrizität das erste Lagerteil in Anlage an das zweite Gegenlagerteil bringbar ist und hierdurch eine Lagerstelle zur Ausbildung eines Schwenkgelenks zwischen den beiden Verlängerungsteilen zur Ausführung der Distraktion oder Kompression gebildet wird.

Es wurde erfindungsgemäß erkannt, dass auf eine aufwändige Kopplungs- und Stellvorrichtung zwischen zwei benachbarten Verlängerungsteilen verzichtet werden kann, indem erfindungsgemäß ein erstes exzentrisches Lagerteil und ein zweites Gegenlagerteil bereitgestellt werden, die insbesondere beide auf die Verlängerungsteile vorzugsweise aufsteckbar oder aufschiebbar sind, wobei das erste exzentrische Lagerteil durch eine einfache Verdrehung relativ zu dem betreffenden Verlängerungsteil in eine berührende Anlage an das Gegenlagerteil bei dem anderen Verlängerungsteil gebracht werden kann. Sobald aber eine solche berührende Anlage gebildet ist, wird hierdurch erfindungsgemäß eine Lagerstelle zur Ausbildung eines Schwenkgelenks zwischen den beiden Verlängerungsteilen zur Ausführung der Distraktion oder Kompression gebildet. Das zweite Gegenlagerteil könnte auch von dem zweiten Verlängerungsteil selbst gebildet sein, indem dort beispielsweise eine Gegenlagerstelle an der Außenoberfläche ausgebildet ist, gegen die sich das erste exzentrische Lagerteil abstützen kann.

Das exzentrische Lagerteil ist so ausgebildet, dass der Radius oder Abstand seines Außenumfangs von der Drehachse (=Längsmittelachse oder -richtung des ersten Verlängerungsteils) in einer Umfangsrichtung zunimmt, so wie wenn ein Körper außerhalb seiner Symmetrieachse drehbar gelagert ist. Das exzentrische Lagerteil ist ferner so ausgebildet, dass eine das Lagerteil außen tangierende einhüllende Kurve in einer Umfangsrichtung einen stetig zunehmenden Radius oder Abstand von der Drehachse aufweist, und zwar zumindest entlang eines Teils des Außenumfangs, der zur Ausbildung einer Lagerstelle mit dem Gegenlagerteil bestimmt ist und ohne in radialer Richtung erstreckte Arme oder dergleichen Fortsätze aufzuweisen.

Es wäre denkbar, dass für die beiden Lagerteile bei dem jeweiligen Verlängerungsteil jeweils eine bestimmte Position in der Längsrichtung zur Positionierung vorgesehen ist. Indessen erweist sich als vorteilhaft, wenn das erste exzentrische Lagerteil und/oder das zweite Gegenlagerteil in Längsrichtung des jeweiligen Verlängerungsteils an variabler Position in der Längsrichtung positionierbar sind, also in der jeweiligen Längsrichtung variabel verstellbar an dem jeweiligen Verlängerungsteil sind. Auf diese Weise kann der Schwenkpunkt oder Gelenkpunkt in der Längsrichtung entsprechend den Gegebenheiten variabel gewählt werden. Es ist lediglich zu berücksichtigen, dass beide Lagerteile auf einander entsprechender Höhe bzw. Position in der Längsrichtung der Verlängerungsteile positioniert werden, damit sie miteinander zur Ausbildung der Lagerstelle zusammenwirken können. Dies wird vom Chirurgen während der Operation dann entsprechend den spezifischen Gegebenheiten ausgewählt. An dem zweiten Verlängerungsteil könnten auch mehrere Gegenlagerstellen in der Längsrichtung aufeinanderfolgend vorgehen sein, um mit dem in Längsrichtung variabel positionierbaren ersten exzentrischen Lagerteil zusammenzuwirken.

Nach einem weiteren Erfindungsgedanken wird vorgeschlagen, dass zwischen dem ersten exzentrischen Lagerteil und dem zweiten Gegenlagerteil in der Längsrichtung formschlüssig hemmende Mittel vorgesehen sind, welche während der Ausführung der Distraktion oder Kompression die Lagerstelle stabilisieren und ein Auseinandergleiten der Lagerteile in der Längsrichtung verhindern. Hierdurch wird die Lagerstelle stabilisiert. Die Formulierung "zwischen dem ersten exzentrischen Lagerteil und dem zweiten Gegenlagerteil" ist hier und im Folgenden in einem funktionellen Sinn zu verstehen; die Mittel können also durch das eine und/oder durch das andere Teil körperlich ausgebildet sein. Dies ist sogar bevorzugt, so dass keine weitere Komponenten Verwendung finden müssen.

Des Weiteren erweist es sich als vorteilhaft, wenn zwischen dem ersten exzentrischen Lagerteil und dem zweiten Gegenlagerteil in der Umfangsrichtung formschlüssig hemmende Mittel vorgesehen sind, welche während der Ausführung der Distraktion oder Kompression die Lagerstelle stabilisieren und ein Auseinandergleiten der Lagerteile in der Umfangsrichtung verhindern. Vorzugsweise ist die Lagerstelle sowohl in der Längsrichtung als auch in der Umfangsrichtung durch solche formschlüssig hemmenden Mittel stabilisiert, um die beiden gegeneinander zu verschwenkenden Verlängerungsteile sicher gegeneinander abzustützen und die Distraktion oder Kompressionsbewegung sicher zu führen.

Die erwähnten formschlüssig hemmenden Mittel könnten in vielfacher Weise, insbesondere durch zusätzliche Eingriffsmittel und Sicherungsmittel in Form von Splinten, Stiften oder dergleichen realisiert werden. Indessen erweist es sich als vorteilhaft, wenn die zwischen dem ersten exzentrischen Lagerteil und dem zweiten Gegenlagerteil in der Längsrichtung formschlüssig hemmenden Mittel und/oder die zwischen dem ersten exzentrischen Lagerteil und dem zweiten Gegenlagerteil in der Umfangsrichtung formschlüssig hemmenden Mittel allein durch eine Oberflächenformgebung des ersten exzentrischen Lagerteils und des zweiten Gegenlagerteils, also ohne weitere Elemente zu erfordern, realisiert ist. Eine derartige Ausbildung vereinfacht die Bedienung und reduziert die Komplexität der Vorrichtung.

In weiterer Ausbildung der Erfindung erweist es sich als vorteilhaft, wenn das erste exzentrische Lagerteil oder das zweite Gegenlagerteil eine in der Umfangsrichtung und insbesondere konzentrisch zur Längsrichtung erstreckte nach radial außen offene Nut und das jeweils andere Lagerteil einen nach radial außen vorstehenden Bereich zum Eingriff in die Nut aufweist. Durch eine derartige Ausbildung lässt sich eine in der Längsrichtung formschlüssige jedoch gleichwohl bewegbare oder schwenkbare Lagefixierung der beiden Lagerteile und damit der beiden Verlängerungsteile zueinander realisieren. Die erwähnte Nut und der erwähnte nach radial außen vorstehende Bereich bilden also in der Längsrichtung formschlüssig hemmende Mittel. Die Breite der Nut ist dabei vorzugsweise größer als die entsprechende Gestaltung des vorstehenden Bereichs zum Eingriff in die Nut, und/oder es ist jedenfalls keine exakt komplementäre Ausbildung gegeben zwischen der Nut und dem vorstehenden Bereichs zum Eingriff in die Nut, damit ein Eingriff und die Ausbildung einer Lagerstelle auch möglich ist, wenn die beiden Verlängerungsteile nicht in einer Ebene sondern insbesondere "windschief" zueinander verlaufen.

Vorzugsweise ist die Nut bei dem zweiten Gegenlagerteil ausgebildet und erstreckt sich vorzugsweise konzentrisch zur Längsrichtung des zugeordneten Verlängerungsteils.

In Weiterbildung der Erfindung erweist es sich als vorteilhaft, wenn die in der Längsrichtung und/oder in der Umfangsrichtung formschlüssig hemmenden Mittel dadurch gebildet sind, dass das erste exzentrische Lagerteil und das zweite Gegenlagerteil im Bereich der Lagerstelle über in der Umfangsrichtung aufeinander folgende Rastpositionen je nach Drehstellung der Lagerteile zueinander gegeneinander anliegen. Nach diesem weiteren Erfindungsgedanken sind in der Umfangsrichtung aufeinanderfolgend diskrete Positionen der beiden Lagerteile relativ zueinander realisiert. Der Begriff Rastposition ist vorliegend im Sinne einer solchen diskreten Anlageposition der beiden Teile zueinander zu verstehen.

Nach einem weiteren Erfindungsgedanken wird vorgeschlagen, dass die in der Längsrichtung und/oder in der Umfangsrichtung formschlüssig hemmenden Mittel dadurch gebildet sind, dass das erste Lagerteil und das zweite Gegenlagerteil im Bereich der Lagerstelle über gekrümmte oder gewölbte Flächenbereiche gegeneinander anliegen. Beispielsweise könnte das eine Lagerteil im Bereich der Lagerstelle eine in Umfangsrichtung erstreckte Nut mit einem insbesondere verrundeten Nutgrund aufweisen, während ein in diese Nut eingreifender radial nach außen vorstehender Bereich am anderen Lagerteil eine hierzu ungefähr komplementäre, insbesondere sattelförmige Gestalt aufweist. Selbstverständlich sind auch andere Ausbildungen denkbar und möglich, wobei eine nur ungefähre komplementäre Ausbildung hinreichend ist, solange ein Eingriff des einen Lagerteils in das andere Lagerteil unter Ausbildung einer lagestabilisierten Lagerstelle ermöglicht ist.

Es wird erfindungsgemäß weiter vorgeschlagen, dass die Rastpositionen oder die gekrümmten oder gewölbten Flächenbereiche durch eine Oberflächengestaltung der beiden Lagerteile und insbesondere durch eine Oberflächengestaltung der Nut am einen Lagerteil und/oder des nach radial außen vorstehenden Bereichs zum Eingriff in die Nut am anderen Lagerteil gebildet ist.

Des Weiteren wird vorgeschlagen, dass das erste exzentrische Lagerteil oder das zweite Gegenlagerteil im Bereich der Anlage an das jeweils andere Lagerteil in der Umfangsrichtung gleichförmig ausgebildet ist und das jeweils andere Lagerteil in der Umfangsrichtung eine variierende, jedoch sich wiederholende Gestaltung aufweist. Auch hierdurch kann eine sich zumindest in der Umfangsrichtung selbsthemmende Ausbildung der Lagerstelle realisiert werden.

Als besonders einfach und daher vorteilhaft erweist es sich, wenn das erste exzentrische Lagerteil und/oder das zweite Gegenlagerteil in Form einer geschlitzten Hülse mit insbesondere im wesentlichen zylindrischer Innenwandung ausgebildet sind/ist. Auf diese Weise können die Lagerteile auf die typischerweise hülsenförmigen Verlängerungsteile aufgesteckt oder aufgeschoben werden.

Hierbei erweist es sich als vorteilhaft, wenn das erste exzentrische Lagerteil und/oder das zweite Gegenlagerteil klemmschlüssig auf dem jeweiligen Verlängerungsteil positionierbar sind/ist. Eine solche klemmschlüssige Positionierbarkeit lässt sich besonders einfach dadurch realisieren, dass ein Innendurchmesser des jeweiligen Lagerteils geringfügig kleiner gewählt wird, als ein Außendurchmesser des betreffenden Verlängerungsteils, wobei das betreffende Lagerteil dann dennoch durch eine geringfügige Aufweitung infolge des Schlitzes auf das betreffende Verlängerungsteil aufschiebbar ist. Die Abmessungen sind dann derart auszuwählen, dass die Kräfte zum Aufstecken oder Aufschieben des jeweiligen Lagerteils leicht manuell aufgebracht werden können, jedoch gleichwohl ein selbsthemmender klemmschlüssiger Sitz des Lagerteils an der vom Chirurgen intendierten axialen Position an dem Verlängerungsteil erzielbar wird.

Weitere Merkmale, Einzelzeiten und Vorteile der Erfindung ergeben sich aus den beigefügten Patentansprüchen und aus der zeichnerischen Darstellung nachfolgenden Beschreibung einer bevorzugten Ausführungsform der Erfindung.

In der Zeichnung zeigt:
Figur 1a eine schematische Darstellung der Verhältnisse bei einer Distraktion von Wirbelkörpern bei einer Wirbelsäulenoperation;
Figur 1b eine schematische Darstellung der Verhältnisse bei einer Kompression von Wirbelkörpern bei einer Wirbelsäulenoperation;
Figur 2 eine perspektivische Darstellung einer erfindungsgemäßen Vorrichtung zum Durchführen einer Distraktion und/oder einer Kompression von Wirbelkörpern bei einer Wirbelsäulenoperation mit zwei Verlängerungsteilen und einem ersten exzentrischen Lagerteil und einem zweiten Gegenlagerteil;
Figur 3 eine perspektivische Darstellung des ersten exzentrischen Lagerteils von Figur 2;
Figur 4 eine teilweise Ansicht des ersten exzentrischen Lagerteils nach Figur 3 in der Längsrichtung von unten betrachtet und
Figur 5 eine perspektivische Ansicht des zweiten Gegenlagerteils.

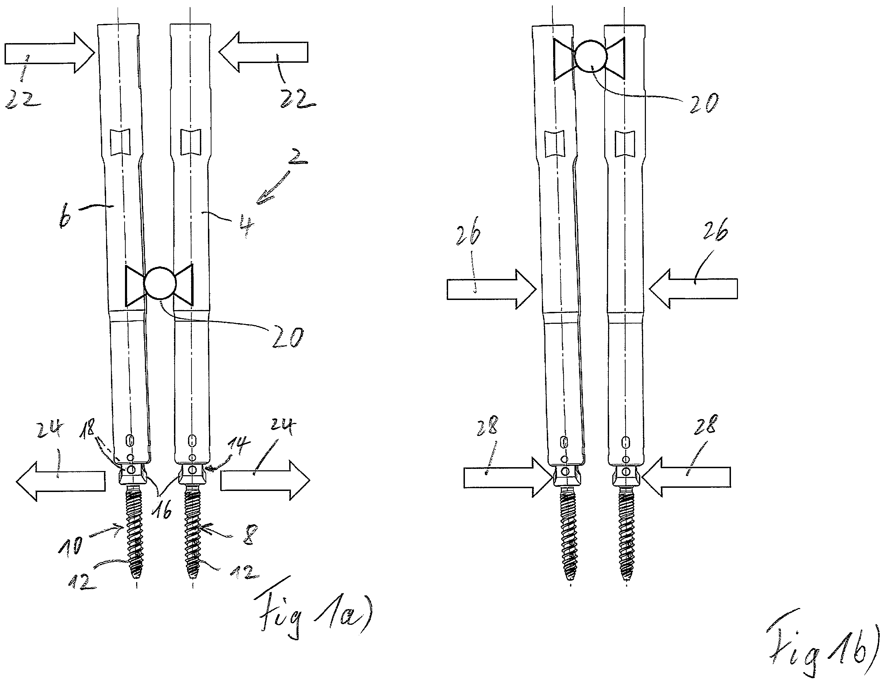

Figur 1a zeigt schematisch die Verhältnisse bei einer Distraktion und Figur 1b bei einer Kompression von Wirbelkörpern bei einer Wirbelsäulenoperation. Dargestellt ist schematisch eine erfindungsgemäße und insgesamt mit dem Bezugszeichen 2 bezeichnete Vorrichtung mit einem ersten und einem zweiten in einem oberen Bereich beispielhaft hülsenförmigen Verlängerungsteil 4 bzw. 6. Das jeweilige Verlängerungsteil 4, 6 ist zwar lösbar jedoch starr und drehfest mit einem zugeordneten Knochenanker 8 bzw. 10 verbunden. Bei dem Knochenanker 8, 10 handelt es sich beispielhaft und vorzugsweise um eine Osteosynthesevorrichtung, wobei der Knochenanker 8, 10 jeweils einen Gewindeschaft 12 zum Einschrauben in einen Wirbelkörper und einen in einer Seitenansicht U-förmigen und eine Aufnahmeöffnung 14 für ein Korrekturelement, insbesondere einen Korrekturstab, aufweisenden Gabelkopf 16 mit zwei Schenkeln 18 umfasst. Der jeweilige Schraubenschaft 12 ist dabei entweder starr, insbesondere einstückig mit dem Gabelkopf 16 ausgebildet, oder er umfasst jeweils einen Kopf, der innerhalb des Gabelkopfs 16 verschwenkbar gelagert ist und in einer intendierten Schwenkstellung bezüglich des Gabelkopfs 16 vom Chirurgen dauerhaft festgelegt wird. Man spricht im letzten Fall von einem sogenannten Polyaxial-Knochenanker, insbesondere einer Polyaxial-Knochenschraube. Nach dem Einbringen, insbesondere Einschrauben der Knochenanker 8, 10 in typischerweise benachbarte Wirbelkörper kann eine Distraktion (Figur 1a) oder eine Kompression (Figur 1b) der Wirbelkörper relativ zueinander ausgeführt werden. Hierfür deutet Figur 1a schematisch eine Lagerstelle 20 nach Art eines Schwenkgelenks zwischen den Verlängerungsteilen 4 und 6 an. Die in Figur 1a oben dargestellten Pfeile 22 deuten eine Krafteinleitung bzw. Schwenkbewegung der beiden Verlängerungsteile 4 und 6 bezüglich der Lagerstelle 20 an. Entsprechend der in Figur 1a unten dargestellten Pfeile 24 werden durch Kraft- und Bewegungsübertragung von den distalen Enden der Verlängerungsteile 4, 6 auf den jeweiligen Gabelkopf 16 des Knochenankers 8 bzw. 10 die Knochenanker 8, 10 und mit ihnen die Wirbelkörper geringfügig voneinander weg bewegt werden, was als Distraktion bezeichnet wird. Im Falle der in Figur 1b angedeuteten Kompression ist eine entsprechend angedeutete Lagerstelle 20 typischerweise in proximaler, also in vom Knochenanker abgewandter Richtung weiter oben vorgesehen, und die distalen Enden der Verlängerungsteile 4, 6 werden bei Krafteinleitung entsprechend der Pfeile 26 aufeinander zubewegt, was durch die Pfeile 28 angedeutet ist. Hierdurch werden die in die Wirbelkörper eingeschraubten Knochenanker 8, 10 und damit die Wirbelkörper aufeinander zubewegt, was als Kompression bezeichnet wird.

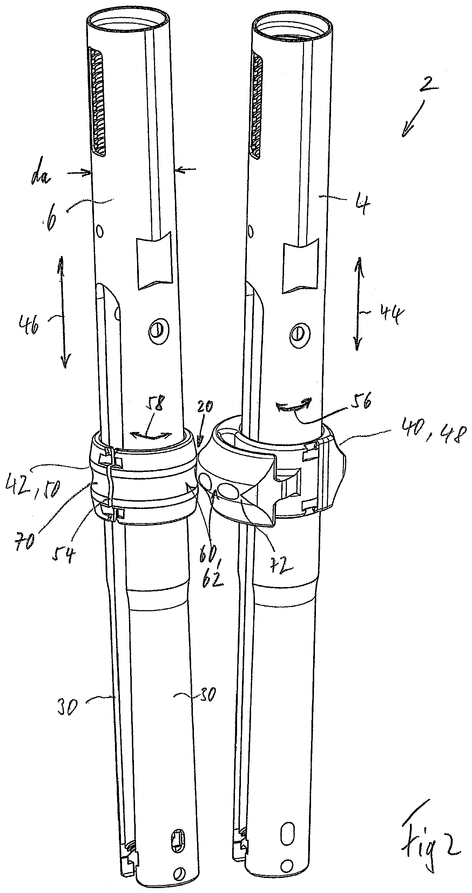

Figur 2 zeigt eine erfindungsgemäße Vorrichtung 2, umfassend zwei Verlängerungsteile 4, 6. Man erkennt, dass im beispielhaft dargestellten Fall die beiden Verlängerungsteile 4, 6 in einem oberen Bereich hülsenförmig ausgebildet sind und dass sie jeweils zwei distal auslaufende, gegeneinander leicht federnde Klammerschenkel 30 aufweisen, mittels derer sie an den Schenkeln 18 des Gabelkopfs 16 eines Knochenankers zwar lösbar, jedoch starr und drehfest fixierbar sind. Zur Fixierung können weitere innerhalb oder außerhalb der Verlängerungsteile 4, 6 zur Wirkung kommende Bestellteile verwendet werden. Anstelle der Klammerschenkel 30 kann auch eine starre Hülsenform mit anderem Befestigungsmechanismus zum Knochenanker hin zum Einsatz kommen. Die erfindungsgemäße Vorrichtung 2 umfasst des Weiteren ein erstes exzentrisches Lagerteil 40 und ein zweites Gegenlagerteil 42. Beide Lagerteile 40 und 42 sind auf das betreffende Verlängerungsteil 4 bzw. 6 aufgesteckt oder aufgeschoben. Sie sind bezüglich des betreffenden Verlängerungsteils 4, 6 in der jeweiligen Längsrichtung 44 bzw. 46 verschiebbar. Sie sind auf einander entsprechender Position in der Längsrichtung 44, 46 positionierbar und im beispielhaft dargestellten Fall klemmschlüssig in dieser Position gehalten.

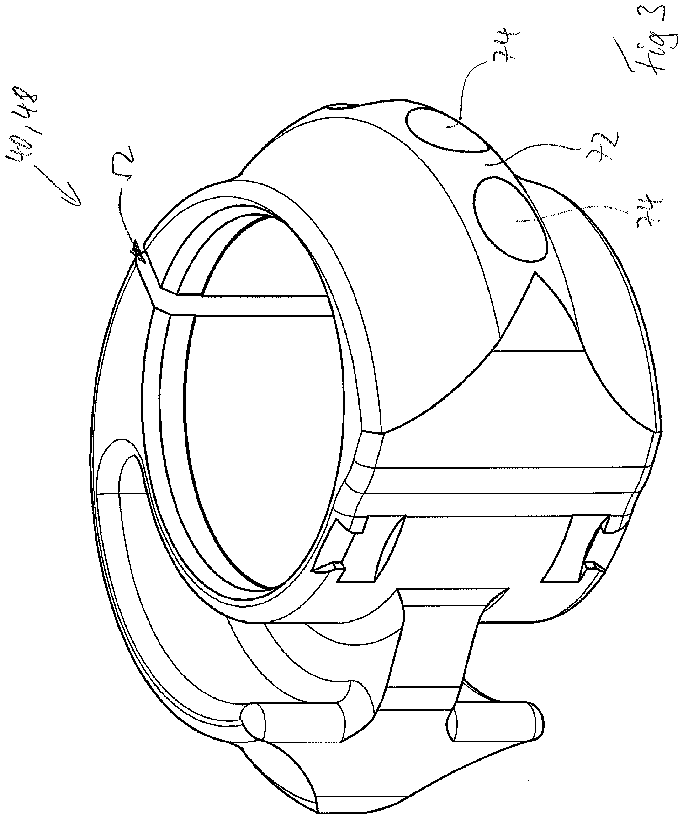

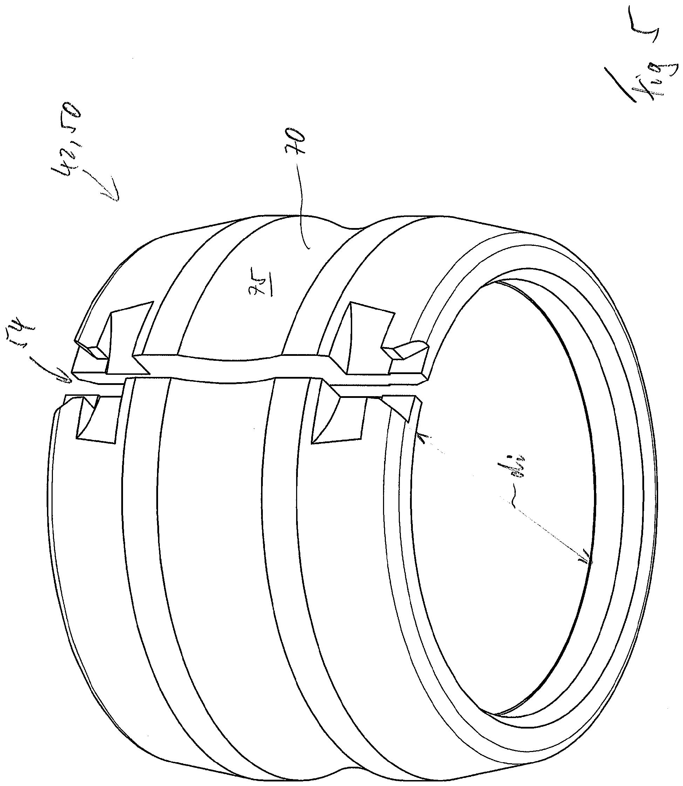

Das erste exzentrische Lagerteil 40 und das zweite Gegenlagerteil 42 sind im dargestellten Fall in Form einer geschlitzten Hülse 48 bzw. 50 ausgebildet. Ein jeweiliger Schlitz 52 bzw. 54 ist auch aus Figuren 3 bis 5 ersichtlich. Eine jeweiliger Inndurchmesser dᵢ einer jeweiligen im wesentlichen zylindrischen Innenwandung der Lagerteile 40, 42 ist im nicht aufgeschobenen Zustand der Lagerteile geringfügigst kleiner als ein Außendurchmesser dₐ der Verlängerungsteile 4, 6. Durch den jeweiligen Schlitz 52, 54 sind die Lagerteile 40, 42 jedoch aufweitbar und lassen sich so auf die Verlängerungsteile 4, 6 in axialer Richtung 44, 46 aufschieben. Sie sind dann klemmschlüssig in einer jeweiligen Position oder Höhe an den Verlängerungsteilen 4, 6 gehalten.

Durch Verdrehen des ersten exzentrischen Lagerteils 40 in einer Umfangsrichtung 56 konzentrisch zur Längsrichtung 44 des betreffenden Verlängerungsteils 4 lässt sich in Folge der Exzentrizität des ersten exzentrischen Lagerteils 40 eine Anlage des ersten exzentrischen Lagerteils 40 mit seinem Außenumfang gegen das zweite Gegenlagerteil 42 realisieren, so wie dies in Figur 2 dargestellt ist. Hierdurch lässt sich die Lagerstelle 20 und mithin die Ausbildung eines Schwenkgelenks zwischen den beiden Verlängerungsteilen 4, 6 realisieren.

Man erkennt am Außenumfang der Lagerteile 40, 42, dass sowohl in der Längsrichtung 44, 46 formschlüssig hemmende Mittel 60 als auch in der Umfangsrichtung 56 formschlüssig hemmende Mittel 62 durch eine entsprechende Gestaltung des Außenumfangs der Lagerteile 40 und 42 realisiert sind.

Im beispielhaft dargestellten Fall umfasst das zweite Gegenlagerteil 42 eine in der Umfangsrichtung 56 konzentrisch zur Längsrichtung 42 erstreckte nach radial außen offene Nut 70. Das erste exzentrische Lagerteil 40 weist einen nach radial außen vorstehenden und in der Umfangsrichtung 56 erstreckten wulstförmigen Bereich 72 zum Eingriff in die Nut 70 auf. Auf diese Weise ist ein formschlüssig wirkendes hemmendes Mittel 60 in der Längsrichtung 44, 46 erreicht, d.h. beim Einleiten von eine Verschwenkung bewirkenden Kräften sind die beiden Lagerteile 40 und 42 in der Längsrichtung gegeneinander gehalten und dadurch die Lagerstelle 20 stabilisiert.

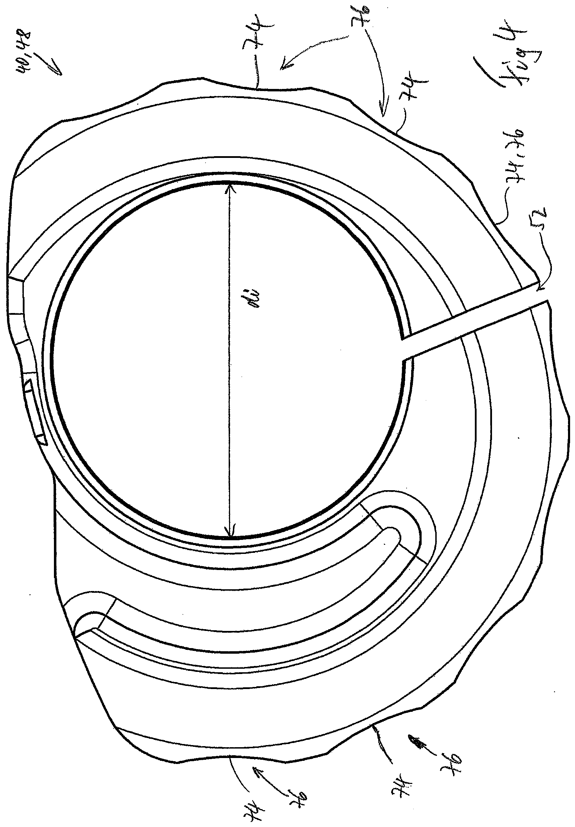

Man erkennt zudem bei dem wulstförmigen Bereich 72 des ersten exzentrischen Lagerteils 40 in der Umfangsrichtung 56 aufeinander folgend angeordnete Flächenbereiche 74 (siehe Figuren 3, 4). Diese Flächenbereiche 74 sind nur ungefähr komplementär und gekrümmt oder gewölbt zu der die Nut 70 begrenzenden Oberfläche 75 des zweiten Gegenlagerteils 42 ausgebildet. Auf diese Weise werden zusätzlich eine Anzahl von in Umfangsrichtung 56 aufeinander folgenden Rastpositionen 76 gebildet, so dass je nach Abstand der Verlängerungsteile 4, 6 voneinander ein geeigneter Flächenbereich 74 zur Ausbildung der Lagerstelle 20 in Kontakt mit der Nut 70 des zweiten Gegenlagerteils 42 gebracht werden kann.

## Patentansprüche

1. Vorrichtung (2) zum Durchführen einer Distraktion und/oder einer Kompression von Wirbelkörpern bei einer, insbesondere minimalinvasiven, Wirbelsäulenoperation, mit einem ersten (4) und einem zweiten (6), insbesondere zumindest abschnittsweise hülsenförmigen, Verlängerungsteil mit einer jeweiligen Längsrichtung (44, 46), welches jeweils an einem Knochenanker (8, 10) zwar lösbar jedoch starr und drehfest festlegbar ist, wobei die Knochenanker (8, 10) jeweils in einen der zu behandelnden Wirbelkörper einzubringen sind, **gekennzeichnet durch** ein erstes exzentrisches Lagerteil (40) und ein zweites Gegenlagerteil (42), die auf einander entsprechender Position in der Längsrichtung (44, 46) an dem ersten (4) bzw. an dem zweiten (6) Verlängerungsteil positionierbar oder positioniert sind, wobei das erste exzentrische Lagerteil (40) ein zusätzliches zu dem ersten Verlängerungsteil (4) bewegbares Bauteil ist und das zweite Gegenlagerteil (42) ein zu dem zweiten Verlängerungsteil (6) zusätzliches bewegbares Bauteil ist oder von dem zweiten Verlängerungsteil (6) selbst gebildet ist, und dadurch dass zumindest das exzentrische Lagerteil (40) relativ zu dem ersten Verlängerungsteil (4) um dessen Längsrichtung (44) drehbar ist, so dass aufgrund der Exzentrizität das erste Lagerteil (40) in Anlage an das zweite Gegenlagerteil (42) bringbar ist und hierdurch eine Lagerstelle (20) zur Ausbildung eines Schwenkgelenks zwischen den beiden Verlängerungsteilen (4, 6) zur Ausführung der Distraktion oder Kompression gebildet wird.

2. Vorrichtung nach Anspruch 1, **dadurch gekennzeichnet, dass** das erste exzentrische Lagerteil (40) und/oder das zweite Gegenlagerteil (42) in Längsrichtung (44, 46) des jeweiligen Verlängerungsteils (4, 6) an variabler Position in der Längsrichtung (44, 46) positionierbar sind.

3. Vorrichtung nach Anspruch 1 oder 2, **dadurch gekennzeichnet, dass** zwischen dem ersten exzentrischen Lagerteil (40) und dem zweiten Gegenlagerteil (42) in der Längsrichtung (44, 46) formschlüssig hemmende Mittel (60) vorgesehen sind, welche während der Ausführung der Distraktion oder Kompression die Lagerstelle (20) stabilisieren und ein Auseinandergleiten der Lagerteile (40, 42) in der Längsrichtung (44, 46) verhindern.

4. Vorrichtung nach Anspruch 1, 2 oder 3, **dadurch gekennzeichnet, dass** zwischen dem ersten exzentrischen Lagerteil (40) und dem zweiten Gegenlagerteil (42) in der Umfangsrichtung (56) formschlüssig hemmende Mittel (62) vorgesehen sind, welche während der Ausführung der Distraktion oder Kompression die Lagerstelle (20) stabilisieren und ein Auseinandergleiten der Lagerteile (40, 42) in der Umfangsrichtung (56) verhindern.

5. Vorrichtung nach Anspruch 3 oder 4, **dadurch gekennzeichnet, dass** die zwischen dem ersten exzentrischen Lagerteil (40) und dem zweiten Gegenlagerteil (42) in der Längsrichtung (44, 46) formschlüssig hemmenden Mittel (60) und/oder die zwischen dem ersten exzentrischen Lagerteil (40) und dem zweiten Gegenlagerteil (42) in der Umfangsrichtung (56) formschlüssig hemmenden Mittel (62) allein durch eine Oberflächenformgebung des ersten exzentrischen Lagerteils (40) und des zweiten Gegenlagerteils (42), also ohne weitere Elemente zu erfordern, realisiert ist.

6. Vorrichtung nach einem oder mehreren der vorstehenden Ansprüche, **dadurch gekennzeichnet, dass** das erste exzentrische Lagerteil (40) oder das zweite Gegenlagerteil (42) eine in der Umfangsrichtung (56, 58) und insbesondere konzentrisch zur Längsrichtung (44, 46) erstreckte nach radial außen offene Nut (70) und das jeweils andere Lagerteil einen nach radial außen vorstehenden Bereich (72) zum Eingriff in die Nut (70) aufweist.

7. Vorrichtung nach Anspruch 3, 4, 5 oder 6, **dadurch gekennzeichnet, dass** die in der Längsrichtung (44, 46) und/oder in der Umfangsrichtung (56) formschlüssig hemmenden Mittel (60, 62) dadurch gebildet sind, dass das erste exzentrische Lagerteil (40) und das zweite Gegenlagerteil (42) im Bereich der Lagerstelle (20) über in der Umfangsrichtung (56) aufeinander folgende Rastpositionen (76) je nach Drehstellung der Lagerteile (40, 42) zueinander gegeneinander anliegen.

8. Vorrichtung nach Anspruch 3, 4, 5, 6 oder 7, **dadurch gekennzeichnet, dass** die in der Längsrichtung (44, 46) und/oder in der Umfangsrichtung (56) formschlüssig hemmenden Mittel (60, 62) dadurch gebildet sind, dass das erste Lagerteil (40) und das zweite Gegenlagerteil (42) im Bereich der Lagerstelle (20) über gekrümmte oder gewölbte Flächenbereiche (74) gegeneinander anliegen.

9. Vorrichtung nach Anspruch 7 oder 8, **dadurch gekennzeichnet, dass** die Rastpositionen oder die gekrümmten oder gewölbten Flächenbereiche (74) durch eine Oberflächengestaltung der Nut (70) und/oder des nach radial außen vorstehenden Bereichs (72) zum Eingriff in die Nut (70) gebildet ist.

10. Vorrichtung nach einem oder mehreren der vorstehenden Ansprüche, **dadurch gekennzeichnet, dass** das erste exzentrische Lagerteil (40) oder das zweite Gegenlagerteil (42) im Bereich der Anlage an das jeweils andere Lagerteil in der Umfangsrichtung (56) gleichförmig ausgebildet ist und das jeweils andere Lagerteil in der Umfangsrichtung (56) eine variierende, jedoch sich wiederholende Gestaltung aufweist.

11. Vorrichtung nach einem oder mehreren der vorstehenden Ansprüche, **dadurch gekennzeichnet, dass** das erste exzentrische Lagerteil (40) und/oder das zweite Gegenlagerteil (42) in Form einer geschlitzten Hülse mit insbesondere im wesentlichen zylindrischer Innenwandung ausgebildet sind/ist.

12. Vorrichtung nach einem oder mehreren der vorstehenden Ansprüche, **dadurch gekennzeichnet, dass** das erste exzentrische Lagerteil (40) und/oder das zweite Gegenlagerteil (42) klemmschlüssig auf dem jeweiligen Verlängerungsteil positionierbar ist.

13. Vorrichtung nach einem oder mehreren der vorstehenden Ansprüche, des Weiteren umfassend je einen Knochenanker (8, 10) für jedes Verlängerungsteil (4, 6), wobei der Knochenanker (8, 10) einen Schaft (12) mit einem Kopf und einen in einer Seitenansicht U-förmigen und eine Aufnahmeöffnung (14) für ein Korrekturelement, insbesondere einen Korrekturstab, aufweisenden Gabelkopf (16) mit zwei Schenkeln (18) umfasst, wobei der Kopf des Schafts (12) und der Gabelkopf (16) entweder miteinander starr, insbesondere einstückig ausgebildet sind oder der Kopf des Schafts (12) an einem distalen Ende des Gabelkopfs (16) verschwenkbar gelagert ist und in diesem Fall der Gabelkopf (16) in einer vom Chirurgen intendierten Schwenkstellung bezüglich des Kopfs des im Knochen festgelegten oder festlegbaren Schafts (12) fixierbar ist.

## Claims

1. Device (2) for carrying out a distraction and/or a compression of vertebral bodies during in particular minimally invasive spinal surgery, said device comprising a first (4) and a second (6) extension part, which parts are in particular sleeve-shaped, at least in portions, each part having a longitudinal direction (44, 46) and each of which parts can be secured on a bone anchor (8, 10) in a detachable but rigid and rotationally fixed manner, the bone anchors (8, 10) in each case being intended to be inserted into one of the vertebral bodies to be treated, **characterized by** a first eccentric bearing part (40) and a second counter bearing part (42), which parts can be positioned or are positioned on the first (4) and on the second (6) extension part respectively in corresponding positions in the longitudinal direction (44,46), the first eccentric bearing part (40) being a movable component that is in addition to the first extension part (4), and the second counter bearing part (42) being a movable component that is in addition to the second extension part (6) or being formed by the second extension part (6) itself, and in that at least the eccentric bearing part (40) can be rotated relative to the first extension part (4) about the longitudinal direction (44) of said extension part, such that due to the eccentricity, the first bearing part (40) can be brought into contact with the second counter bearing part (42) and thus a bearing point (20) is formed for forming a pivot joint between the two extension parts (4, 6) in order to perform the distraction or compression.

2. Device according to claim 1, **characterized in that**, in the longitudinal direction (44, 46) of the relevant extension part (4, 6), the first eccentric bearing part (40) and/or the second counter bearing part (42) can be positioned in variable positions in the longitudinal direction (44, 46).

3. Device according to claim 1 or claim 2, **characterized in that** means (60) that are positively arresting in the longitudinal direction (44, 46) are provided between the first eccentric bearing part (40) and the second counter bearing part (42), which means stabilize the bearing point (20) and prevent the bearing parts (40, 42) from sliding apart in the longitudinal direction (44, 46) while the distraction or compression is being performed.

4. Device according to claim 1, 2 or 3, **characterized in that** means (62) that are positively arresting in the peripheral direction (56) are provided between the first eccentric bearing part (40) and the second counter bearing part (42), which means stabilize the bearing point (20) and prevent the bearing parts (40, 42) from sliding apart in the peripheral direction (56) while the distraction or compression is being performed.

5. Device according to claim 3 or claim 4, **characterized in that** the means (60) that are positively arresting in the longitudinal direction (44, 46) and that are provided between the first eccentric bearing part (40) and the second counter bearing part (42) and/or the means (62) that are positively arresting in the peripheral direction (56) and that are provided between the first eccentric bearing part (40) and the second counter bearing part (42) are formed simply by a surface shaping of the first eccentric bearing part (40) and of the second counter bearing part (42), i.e. without requiring further elements.

6. Device according to one or more of the preceding claims, **characterized in that** the first eccentric bearing part (40) or the second counter bearing part (42) comprises a radially outwardly open groove (70) that extends in the peripheral direction (56, 58) and in particular concentrically to the longitudinal direction (44, 46), and the other bearing part in each case comprises a radially outwardly projecting region (72) for engaging in the groove (70).

7. Device according to claim 3, 4, 5 or 6, **characterized in that** the means (60, 62) that are positively arresting in the longitudinal direction (44, 46) and/or in the peripheral direction (56) are formed by the first eccentric bearing part (40) and the second counter bearing part (42) being in contact with one another, depending on the rotational position of the bearing parts (40, 42) with respect to one another, in the region of the bearing point (20) by means of locking positions (76) that are in succession in the peripheral direction (56).

8. Device according to claim 3, 4, 5, 6 or 7, **characterized in that** the means (60, 62) that are positively arresting in the longitudinal direction (44, 46) and/or in the peripheral direction (56) are formed by the first bearing part (40) and the second counter bearing part (42) being in contact with one another over curved or rounded surface regions (74) in the region of the bearing point (20) .

9. Device according to claim 7 or claim 8, **characterized in that** the locking position or the curved or rounded surface regions (74) is/are formed by a surface shape of the groove (70) and/or of the radially outwardly projecting region (72) for engaging in the groove (70).

10. Device according to one or more of the preceding claims, **characterized in that** the first eccentric bearing part (40) or the second counter bearing part (42) is formed homogenously in the peripheral direction (56) in the region of the contact with the other bearing part in each case, and the other bearing part in each case has a shape in the peripheral direction (56) that varies but repeats.

11. Device according to one or more of the preceding claims, **characterized in that** the first eccentric bearing part (40) and/or the second counter bearing part (42) is formed as a slotted sleeve comprising in particular a substantially cylindrical inside wall.

12. Device according to one or more of the preceding claims, **characterized in that** the first eccentric bearing part (40) and/or the second counter bearing part (42) can be positioned on the relevant extension part in a clamped manner.

13. Device according to one or more of the preceding claims, further comprising one bone anchor (8, 10) in each case for each extension part (4, 6), the bone anchor (8, 10) comprising a shank (12) having a head, and a clevis (16) that is U-shaped in a side view, has a receiving opening (14) for a correction element, in particular a correction rod, and has two limbs (18), the head of the shank (12) and the clevis (16) either being rigidly interconnected, in particular integral, or the head of the shank (12) being pivotally mounted on a distal end of the clevis (16) and in this case the clevis (16) being able to be fixed in a pivot position intended by the surgeon relative to the head of the shank (12) that is secured or can be secured in the bone.

## Revendications

1. Dispositif (2) de mise en oeuvre d'une distraction et/ou d'une compression de corps vertébraux dans une intervention chirurgicale sur la colonne vertébrale, en particulier mini-invasive, comprenant une première (4) et une deuxième (6) partie d'allongement qui est en forme de douille, en particulier au moins par sections, et présente une direction longitudinale (44, 46) respective et qui peut être fixée respectivement sur un ancre à os (8, 10), certes, de manière amovible, mais rigide et solidaire en rotation, dans lequel les ancres à os (8, 10) sont à implanter chacun dans un des corps vertébraux à traiter, **caractérisé par** une première partie de support (40) excentrique et une deuxième contre-partie de support (42) qui peuvent être positionnées ou sont positionnées sur ladite première (4) ou bien ladite deuxième (6) partie d'allongement dans une position correspondant l'une à l'autre dans la direction longitudinale (44, 46), dans lequel la première partie de support (40) excentrique est un composant supplémentaire déplaçable par rapport à la première partie d'allongement (4) et ladite deuxième contre-partie de support (42) est un composant supplémentaire déplaçable par rapport à la deuxième partie d'allongement (6) ou est formée par la deuxième partie d'allongement (6) même, et par le fait qu'au moins la partie de support (40) excentrique peut tourner par rapport à la première partie d'allongement (4) autour de la direction longitudinale (44) de celle-ci de sorte que, en raison de l'excentricité, la première partie de support (40) peut être mise en appui sur la deuxième contre-partie de support (42) et que, ainsi, un point de support (20) est formé pour la réalisation d'une articulation de pivotement entre les deux parties d'allongement (4, 6) afin de mettre en oeuvre la distraction ou la compression.

2. Dispositif selon la revendication 1, **caractérisé par le fait que** la première partie de support (40) excentrique et/ou la deuxième contre-partie de support (42) peuvent être positionnées dans la direction longitudinale (44, 46) de la partie d'allongement (4, 6) respective sur une position variable dans la direction longitudinale (44, 46).

3. Dispositif selon la revendication 1 ou 2, **caractérisé par le fait que** des moyens (60) qui bloquent à engagement positif dans la direction longitudinale (44, 46) sont prévus entre la première partie de support (40) excentrique et la deuxième contre-partie de support (42), ceux-ci stabilisent ledit point de support (20) lorsque la distraction ou la compression est réalisée et empêchent que les parties de support (40, 42) ne s'écartent l'une de l'autre en glissant dans la direction longitudinale (44, 46).

4. Dispositif selon la revendication 1, 2 ou 3, **caractérisé par le fait que** des moyens (62) qui bloquent à engagement positif dans la direction circonférentielle (56) sont prévus entre la première partie de support (40) excentrique et la deuxième contre-partie de support (42), ceux-ci stabilisent ledit point de support (20) lorsque la distraction ou la compression est réalisée et empêchent que les parties de support (40, 42) ne s'écartent l'une de l'autre en glissant dans la direction circonférentielle (56).

5. Dispositif selon la revendication 3 ou 4, **caractérisé par le fait que** lesdits moyens (60) bloquant à engagement positif dans la direction longitudinale (44, 46) qui sont prévus entre la première partie de support (40) excentrique et la deuxième contre-partie de support (42), et/ou lesdits moyens (62) bloquant à engagement positif dans la direction circonférentielle (56) qui sont prévus entre la première partie de support (40) excentrique et la deuxième contre-partie de support (42) sont réalisés uniquement par une conformation de surface de la première partie de support (40) excentrique et de la deuxième contre-partie de support (42), donc sans requérir d'autres éléments.

6. Dispositif selon l'une ou plusieurs des revendications précédentes, **caractérisé par le fait que** la première partie de support (40) excentrique ou la deuxième contre-partie de support (42) présente une rainure (70) ouverte radialement vers l'extérieur qui s'étend dans la direction circonférentielle (56, 58) et, en particulier, est concentrique à la direction longitudinale (44, 46), et l'autre partie de support respective présente une partie (72) faisant radialement saillie vers l'extérieur et destinée à s'engager dans ladite rainure (70).

7. Dispositif selon la revendication 3, 4, 5 ou 6, **caractérisé par le fait que** lesdits moyens (60, 62) bloquant à engagement positif dans la direction longitudinale (44, 46) et/ou dans la direction circonférentielle (56) sont réalisés **par le fait que** la première partie de support (40) excentrique et la deuxième contre-partie de support (42) s'appliquent l'une contre l'autre au niveau du point de support (20) par des positions d'arrêt (76) successives dans la direction circonférentielle (56), selon la position de rotation des parties de support (40, 42) l'une par rapport à l'autre.

8. Dispositif selon la revendication 3, 4, 5, 6 ou 7, **caractérisé par le fait que** lesdits moyens (60, 62) bloquant à engagement positif dans la direction longitudinale (44, 46) et/ou dans la direction circonférentielle (56) sont réalisés **par le fait que** la première partie de support (40) et la deuxième contre-partie de support (42) s'appliquent l'une contre l'autre au niveau du point de support (20) par des zones de surface (74) courbées ou bombées.

9. Dispositif selon la revendication 7 ou 8, **caractérisé par le fait que** les positions d'arrêt ou les zones de surface (74) courbées ou bombées sont réalisées par une conformation de surface de la rainure (70) et/ou de la zone (72) faisant saillie radialement vers l'extérieur et destinée à s'engager dans la rainure (70).

10. Dispositif selon l'une ou plusieurs des revendications précédentes, **caractérisé par le fait que**, au niveau de l'appui sur l'autre partie de support respective, la première partie de support (40) excentrique ou la deuxième contre-partie de support (42) est réalisée de manière uniforme dans la direction circonférentielle (56) et l'autre partie de support respective présente une conformation variante, mais se répétant, dans la direction circonférentielle (56).

11. Dispositif selon l'une ou plusieurs des revendications précédentes, **caractérisé par le fait que** la première partie de support (40) excentrique et/ou la deuxième contre-partie de support (42) est/sont réalisée(s) sous la forme d'une douille fendue ayant une paroi intérieure en particulier pour l'essentiel cylindrique.

12. Dispositif selon l'une ou plusieurs des revendications précédentes, **caractérisé par le fait que** la première partie de support (40) excentrique et/ou la deuxième contre-partie de support (42) peut être positionnée par liaison par serrage sur la partie d'allongement respective.

13. Dispositif selon l'une ou plusieurs des revendications précédentes, comprenant en outre respectivement un ancre à os (8, 10) pour chaque partie d'allongement (4, 6), dans lequel l'ancre à os (8, 10) comprend une tige (12) ayant une tête ainsi qu'une tête à fourche (16) en U, vue de côté, qui présente un orifice de logement (14) pour un élément de correction, en particulier une barre de correction, et comprend deux branches (18), dans lequel ladite tête de la tige (12) et ladite tête à fourche (16) sont réalisées soit rigidement l'une avec l'autre, en particulier d'un seul tenant, ou la tête de la tige (12) est logée à pivotement à une extrémité distale de la tête à fourche (16) et, dans ce cas, la tête à fourche (16) peut être fixée dans une position de pivotement, envisagée par le chirurgien, par rapport à la tête de la tige (12) fixée ou apte à être fixée dans l'os.
